# EUROPEAN PATENT APPLICATION

(11) **EP 4 439 569 A1**
(43) Date of publication of application: **02.10.2024**
(21) Application number: 22898464.7
(22) Date of filing: 15.11.2022
(51) Int. Cl.: G16C 20/30, G16C 20/70, G06N 3/02

(54) **PREDICTION DEVICE, LEARNING DEVICE, PREDICTION METHOD, LEARNING METHOD, PREDICTION PROGRAM, AND LEARNING PROGRAM**

(30) Priority: 24.11.2021 JP 2021189833
(71) Applicant: Resonac Corporation, Tokyo 105-7325 (JP)
(72) Inventor: OKANO, Yu, Tokyo 105-8518 (JP); KANESHITA, Takeshi, Tokyo 105-8518 (JP); TAKEMOTO, Shimpei, Tokyo 105-8518 (JP); OKUNO, Katsuki, Tokyo 105-8518 (JP); OKUNO, Yoshishige, Tokyo 105-8518 (JP)
(74) Representative: Mewburn Ellis LLP
(86) International application number: PCT/JP2022/042403
(87) International publication number: WO 2023/095680

(57) **Abstract**

To improve prediction accuracy in a prediction model that predicts a phase fraction over a predetermined temperature range based on a material composition. A prediction apparatus includes a trained model trained by using training data in which a material composition of a material to be learned is associated with a phase fraction of the material to be learned at each temperature within a predetermined temperature range, the trained model being configured to predict a phase fraction at an i+1-th temperature by using a phase fraction predicted by the trained model for one or more temperatures up to an i-th temperature within the predetermined temperature range (where i is an integer of 1 or more). The prediction apparatus is configured to input a material composition of a material to be predicted into the trained model, thereby predicting a phase fraction of the material to be predicted at each temperature within the predetermined temperature range.

## Description

### Technical Field

The present disclosure relates to a prediction apparatus, a training apparatus, a prediction method, a training method, a prediction program, and a training program.

### Background Art

In the design of materials such as alloys, it is important to calculate the phase fraction at thermodynamic equilibrium for a predetermined temperature range, and models are being developed to calculate the phase fraction based on material composition information at low cost.

As an example, Patent Document 1 discloses a model for predicting the phase fraction at each temperature within a predetermined temperature range at low cost by using a multilayer neural network.

### RELATED-ART DOCUMENT

### Patent Document

Patent Document 1: International Publication No. WO 2020/090617

### Summary of Invention

### Technical Problem

However, in the case of the model using the multilayer neural network, when calculating the phase fraction at each temperature, a prediction result of the phase fraction in the adjacent temperature region is not reflected. Therefore, when the phase fraction is predicted for a predetermined temperature range, prediction accuracy of the phase fraction may be reduced in some temperature regions.

The object of the present disclosure is to improve the prediction accuracy in a model that predicts the phase fraction over a predetermined temperature range based on a material composition.

### Solution to Problem

A first aspect of the present disclosure is a prediction apparatus including:
a trained model trained by using training data in which a material composition of a material to be learned is associated with a phase fraction of the material to be learned at each temperature within a predetermined temperature range, the trained model being configured to predict a phase fraction at an i+1-th temperature by using a phase fraction predicted by the trained model for one or more temperatures up to an i-th temperature within the predetermined temperature range (where i is an integer of 1 or more), wherein
the prediction apparatus is configured to input a material composition of a material to be predicted into the trained model, thereby predicting a phase fraction of the material to be predicted at each temperature within the predetermined temperature range.

A second aspect of the present disclosure is the prediction apparatus according to the first aspect, wherein the trained model is applied with an architecture capable of calculating time series data that is data for each of predetermined time intervals, and the trained model predicts a phase fraction for each of predetermined temperature intervals based on the material composition of the material to be predicted.

A third aspect of the present disclosure is the prediction apparatus according to the second aspect, wherein the trained model is any one of RNN, Bidirectional RNN, Seq2Seq, Seq2Seq with Attention mechanism, GRU, LSTM, or Transformer.

A fourth aspect of the present disclosure is the prediction apparatus according to the third aspect, wherein the trained model includes:
an encoder configured to output a feature, upon input of the material composition of the material to be predicted; and
a decoder configured to predict the phase fraction at the i+1-th temperature, upon input of the output feature and the phase fraction predicted for the temperature up to the i-th temperature.

A fifth aspect of the present disclosure is the prediction apparatus according to the first aspect, wherein the phase fraction is a phase fraction at thermodynamic equilibrium.

A sixth aspect of the present disclosure is a training apparatus including:
a model trained by using training data in which a material composition of a material to be learned is associated with a phase fraction of the material to be learned at each temperature within a predetermined temperature range, the model being configured to output output data corresponding to a phase fraction at an i+1-th temperature by using ground truth data for one or more temperatures up to an i-th temperature within the predetermined temperature range (where i is an integer of 1 or more).

A seventh aspect of the present disclosure is the training apparatus according to the sixth aspect, wherein the model includes:
an encoder configured to output a feature, upon input of the material composition of the material to be learned; and
a decoder configured to output the output data corresponding to the phase fraction at the i+1-th temperature, upon input of the output feature and the ground truth data of the phase fraction for the temperature up to the i-th temperature.

An eighth aspect of the present disclosure is the training apparatus according to the sixth aspect, further comprising a calculation unit configured to, upon input of the material composition of the material to be learned, compare the output data output by the model with the phase fraction at each temperature within the predetermined temperature range that is associated with the material composition of the material to be learned, thereby calculating a loss function.

A ninth aspect of the present disclosure is the training apparatus according to the eighth aspect, wherein the calculation unit is configured to calculate the loss function and to output a loss, and the loss includes at least any one of:
a first addition result obtained by adding, for all phases, an error between a phase fraction specified based on the output data and a phase fraction specified based on the training data, at a temperature at which each phase is formed or disappeared, the temperature being specified based on the training data;
a second addition result obtained by adding, for the predetermined temperature range, an error between a phase fraction at each temperature included in the output data and a phase fraction at each temperature included in the training data;
a third addition result obtained by adding, for the predetermined temperature range, an error between a logarithmic value of the phase fraction at each temperature included in the output data and a logarithmic value of the phase fraction at each temperature included in the training data;
a fourth addition result obtained by adding, for the predetermined temperature range, an error between a differential value of phase fractions between adjacent temperatures among a group of the phase fraction at each temperature included in the output data and a differential value of the phase fractions between adjacent temperatures among a group of the phase fraction at each temperature included in the training data; or
a fifth addition result obtained by adding, for the predetermined temperature range, an error between a ratio of the phase fraction at each temperature included in the output data and a ratio of the phase fraction at each temperature included in the training data.

A tenth aspect of the present disclosure is the training apparatus according to the ninth aspect, wherein the calculation unit is configured to perform a weighted addition of the first addition result to the fifth addition result.

An eleventh aspect of the present disclosure is the training apparatus according to the ninth aspect, wherein when calculating the logarithmic value of the phase fraction, the calculation unit is configured to add a value according to a decimal place of the phase fraction, thereby making the logarithmic value of the phase fraction non-negative.

A twelfth aspect of the present disclosure is the training apparatus according to the eighth aspect, wherein the calculation unit is configured to divide the predetermined temperature range into a specific range including a plurality of phase formations and a plurality of phase disappearances and into a non-specific range, which is a range excluding the specific range, thereby calculating a loss function for the specific range and a loss function for the non-specific range separately.

A thirteenth aspect of the present disclosure is the training apparatus according to the sixth aspect, wherein the material composition of the material to be learned included in the training data is determined by an amount selected at random between a lower limit and an upper limit of an amount of each element.

A fourteenth aspect of the present disclosure is the training apparatus according to the sixth aspect, wherein the material composition includes any one of a chemical composition indicating a ratio of each chemical component contained in a material; or an alloy composition indicating a ratio of each metallic element or each non-metallic element included in an alloy.

A fifteenth aspect of the present disclosure is a prediction method including:
executing, by a computer, a trained model trained by using training data in which a material composition of a material to be learned is associated with a phase fraction of the material to be learned at each temperature within a predetermined temperature range, the trained model being configured to predict a phase fraction at an i+1-th temperature by using a phase fraction predicted by the trained model for one or more temperatures up to an i-th temperature within the predetermined temperature range (where i is an integer of 1 or more), wherein
a material composition of a material to be predicted is input into the trained model, thereby a phase fraction of the material to be predicted is predicted at each temperature within the predetermined temperature range.

A sixteenth aspect of the present disclosure is a training method including: executing, by a computer, a model trained by using training data in which a material composition of a material to be learned is associated with a phase fraction of the material to be learned at each temperature within a predetermined temperature range, the model being configured to output output data corresponding to a phase fraction at an i+1-th temperature by using ground truth data for one or more temperatures up to an i-th temperature within the predetermined temperature range (where i is an integer of 1 or more).

A seventeenth aspect of the present disclosure is a prediction program for causing a computer to execute a trained model trained by using training data in which a material composition of a material to be learned is associated with a phase fraction of the material to be learned at each temperature within a predetermined temperature range, the trained model being configured to predict a phase fraction at an i+1-th temperature by using a phase fraction predicted by the trained model for one or more temperatures up to an i-th temperature within the predetermined temperature range (where i is an integer of 1 or more), wherein
a material composition of a material to be predicted is input into the trained model, thereby a phase fraction of the material to be predicted is predicted at each temperature within the predetermined temperature range.

An eighteenth aspect of the present disclosure is a training program for causing a computer to execute a model trained by using training data in which a material composition of a material to be learned is associated with a phase fraction of the material to be learned at each temperature within a predetermined temperature range, the model being configured to output output data corresponding to a phase fraction at an i+1-th temperature by using ground truth data for one or more temperatures up to an i-th temperature within the predetermined temperature range (where i is an integer of 1 or more).

### Advantageous Effects of Invention

According to the present disclosure, prediction accuracy can be improved in a model that predicts a phase fraction over a predetermined temperature range based on a material composition.

### Brief Description of the Drawings

[FIG. 1]

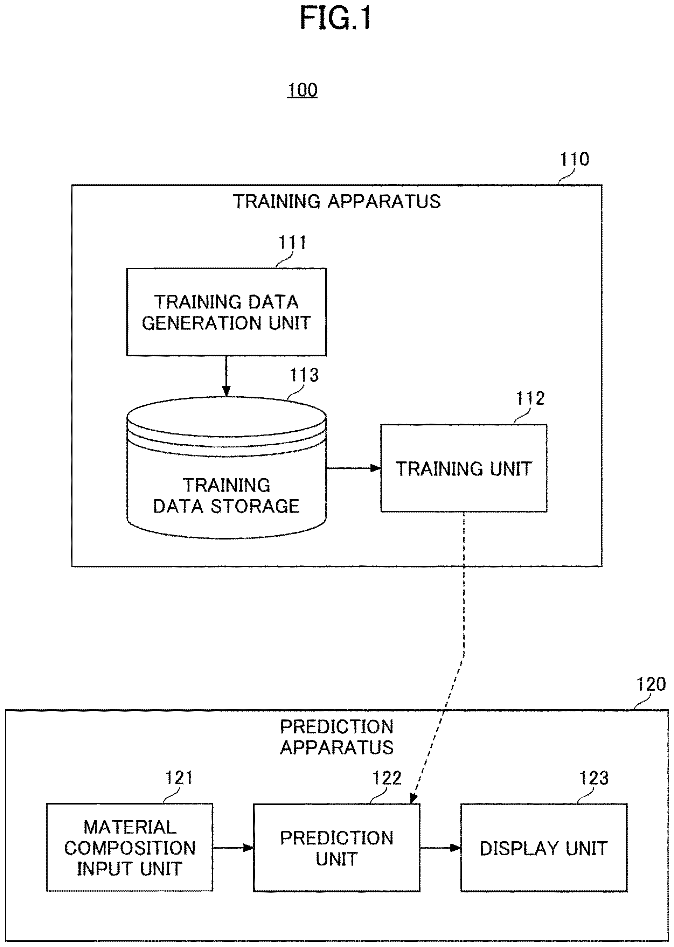

   FIG. 1 is a diagram illustrating an example of a system configuration of a prediction system and an example of a functional configuration of a training apparatus and a prediction apparatus.
[FIG. 2]

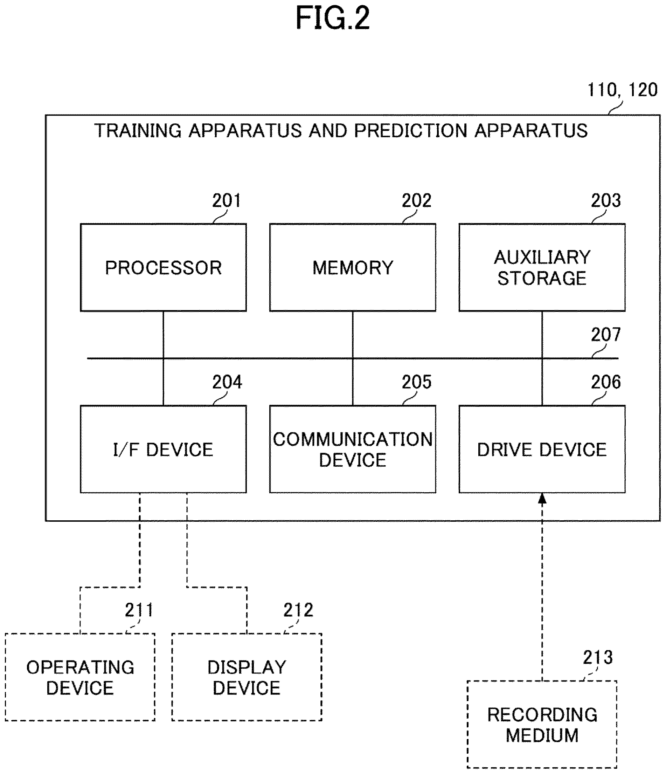

   FIG. 2 is a diagram illustrating an example of a hardware configuration of the training apparatus and the prediction apparatus.
[FIG. 3]

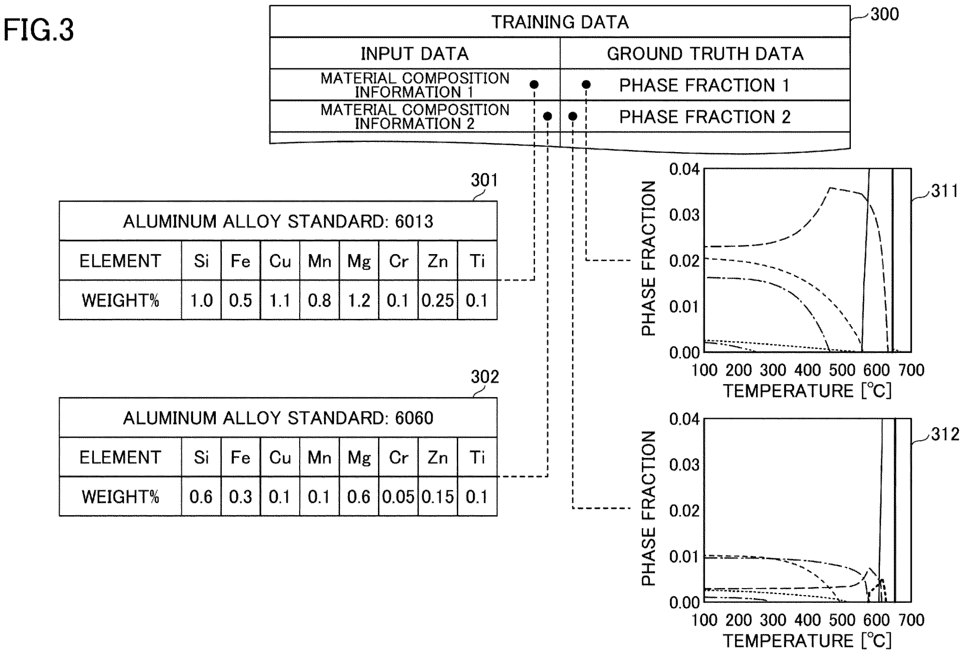

   FIG. 3 is a diagram illustrating a configuration of training data and specific examples of input data and ground truth data.
[FIG. 4]

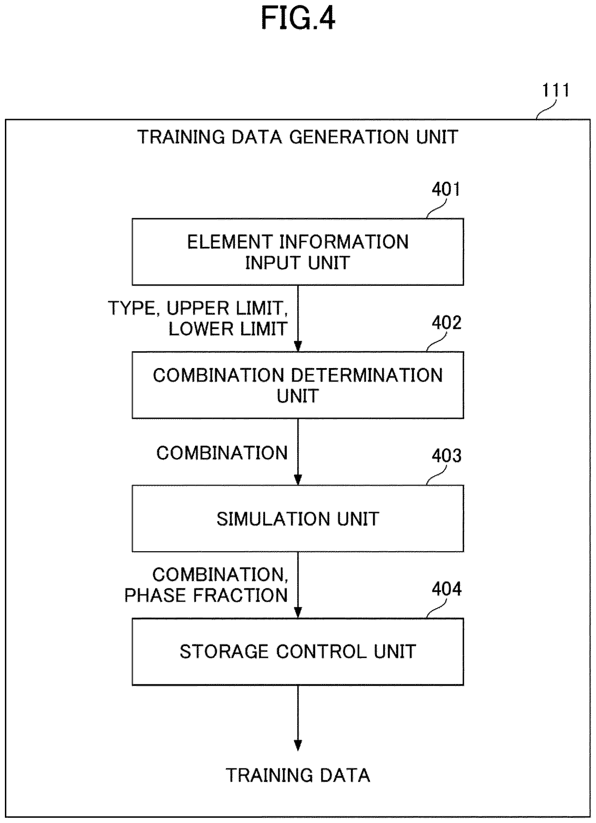

   FIG. 4 is a diagram illustrating an example of a functional configuration of a training data generation unit.
[FIG. 5]

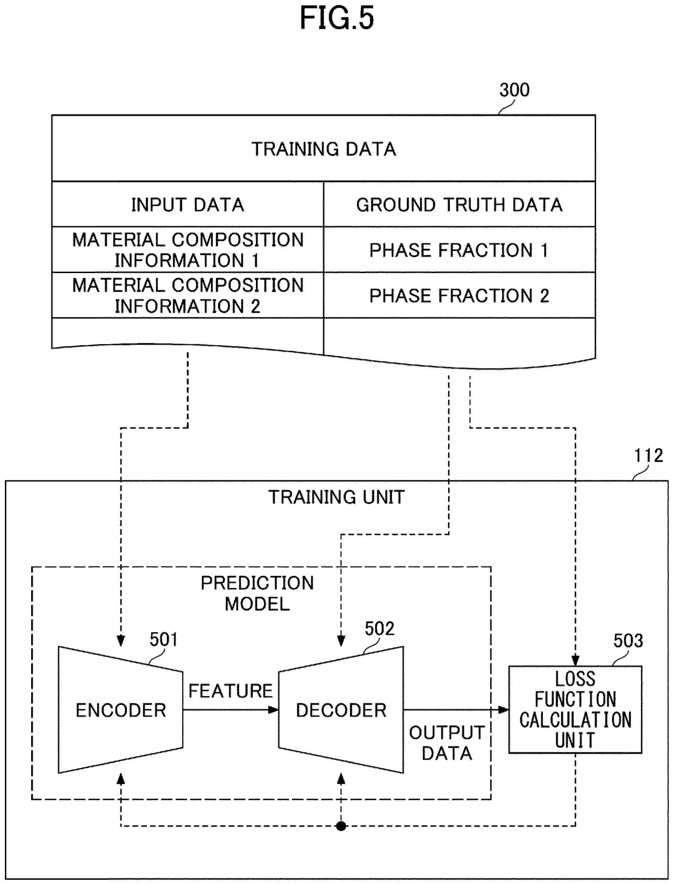

   FIG. 5 is a diagram illustrating an example of a functional configuration of a training unit.
[FIG. 6]

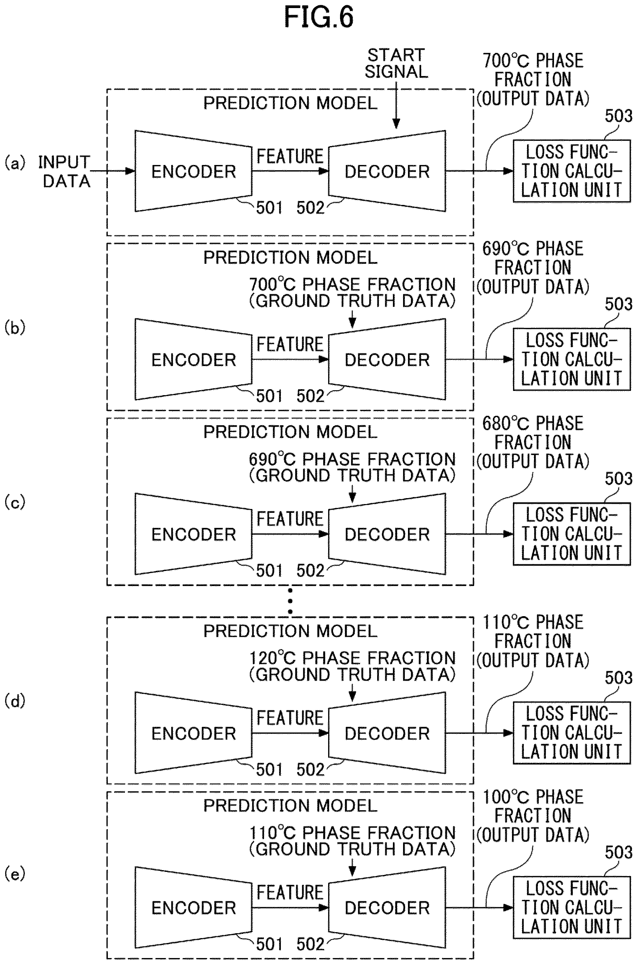

   FIG. 6 is a first diagram illustrating an operation example of the training unit.
[FIG. 7]

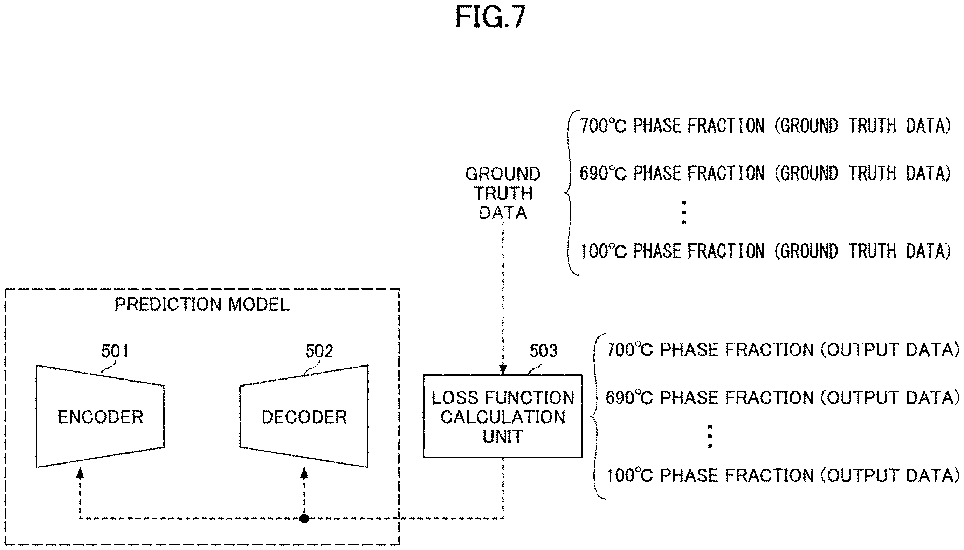

   FIG. 7 is a second diagram illustrating an operation example of the training unit.
[FIG. 8]

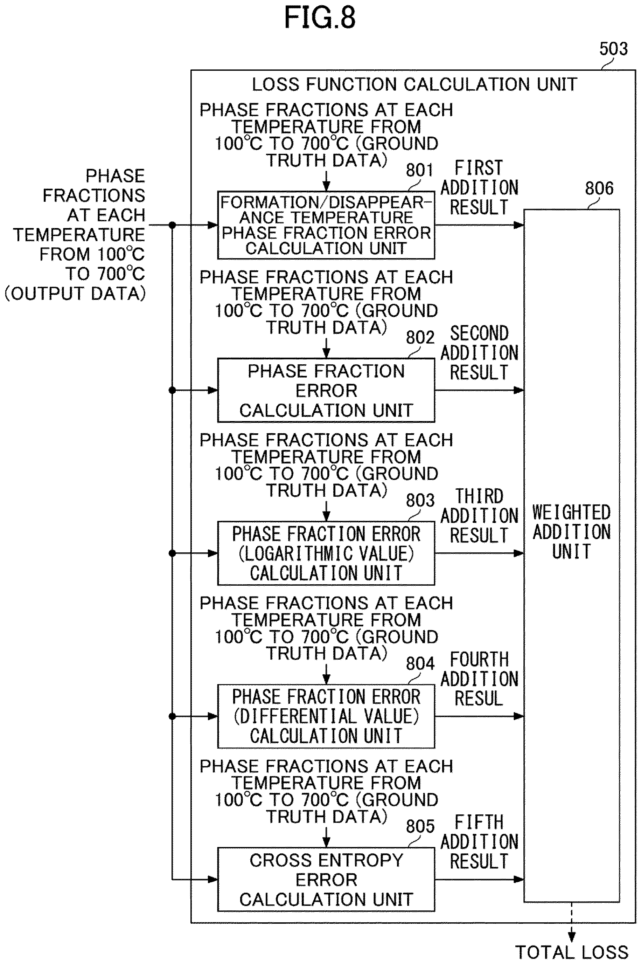

   FIG. 8 is a first diagram illustrating an example of a loss calculation method by a loss function calculation unit.
[FIG. 9]

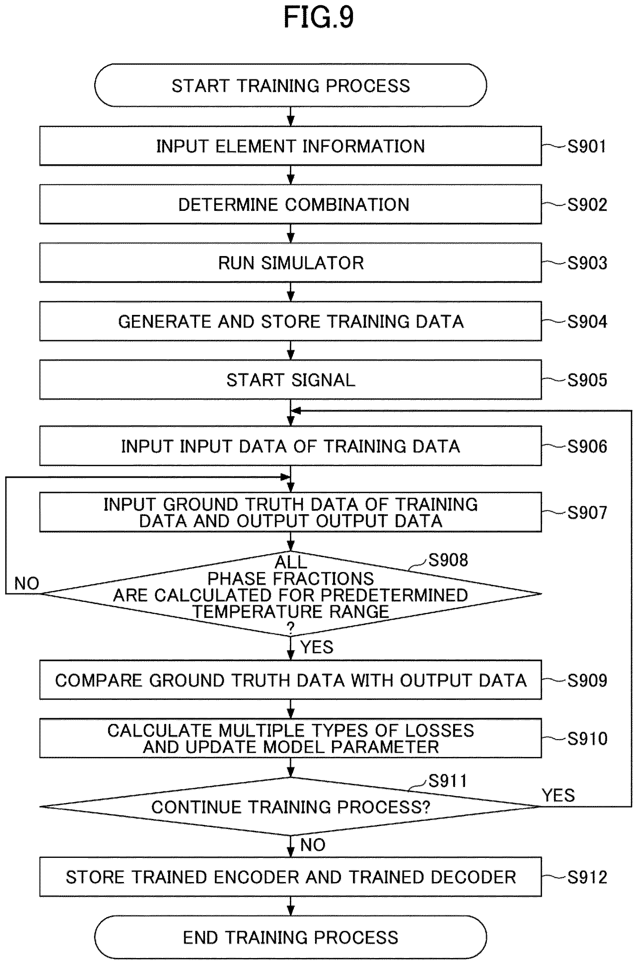

   FIG. 9 is a flowchart illustrating a flow of a training process.
[FIG. 10]

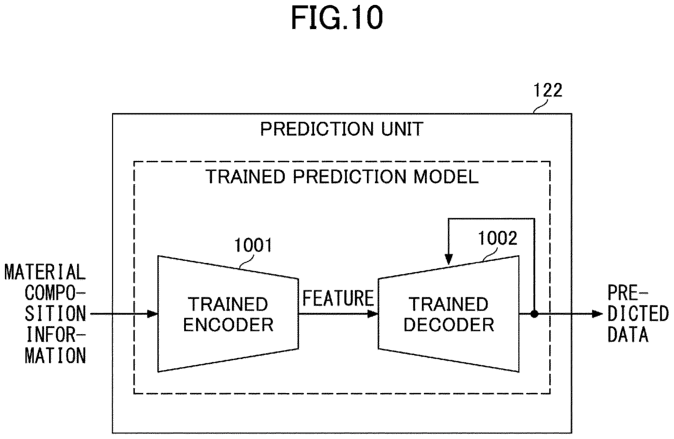

   FIG. 10 is a diagram illustrating an example of a functional configuration of a prediction unit.
[FIG. 11]

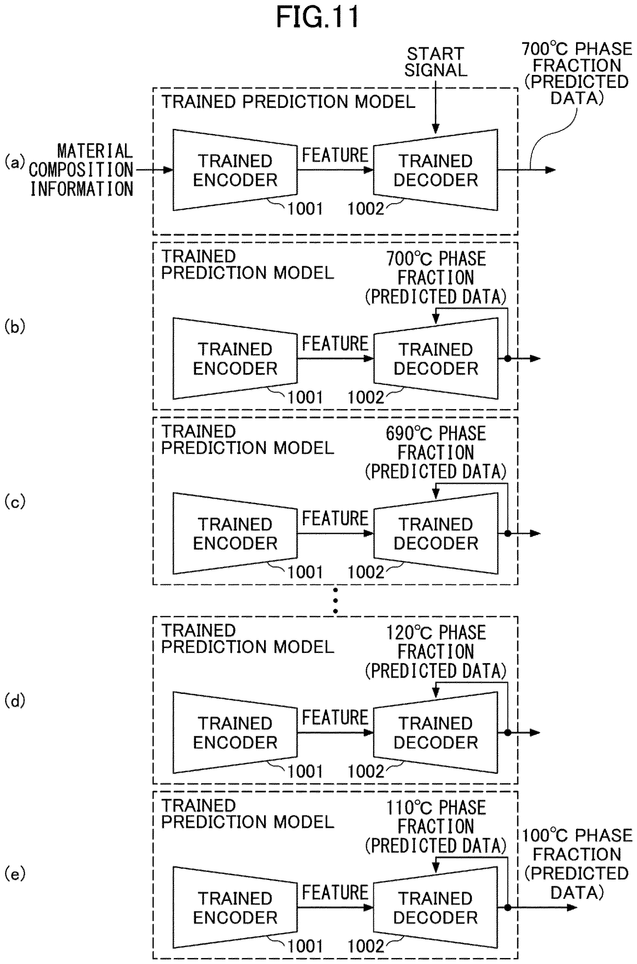

   FIG. 11 is a diagram illustrating an operation example of the prediction unit.
[FIG. 12]

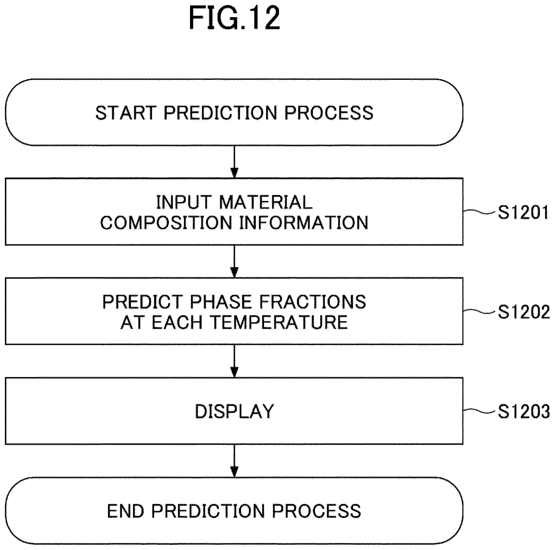

   FIG. 12 is a flowchart illustrating a flow of a prediction process.
[FIG. 13]

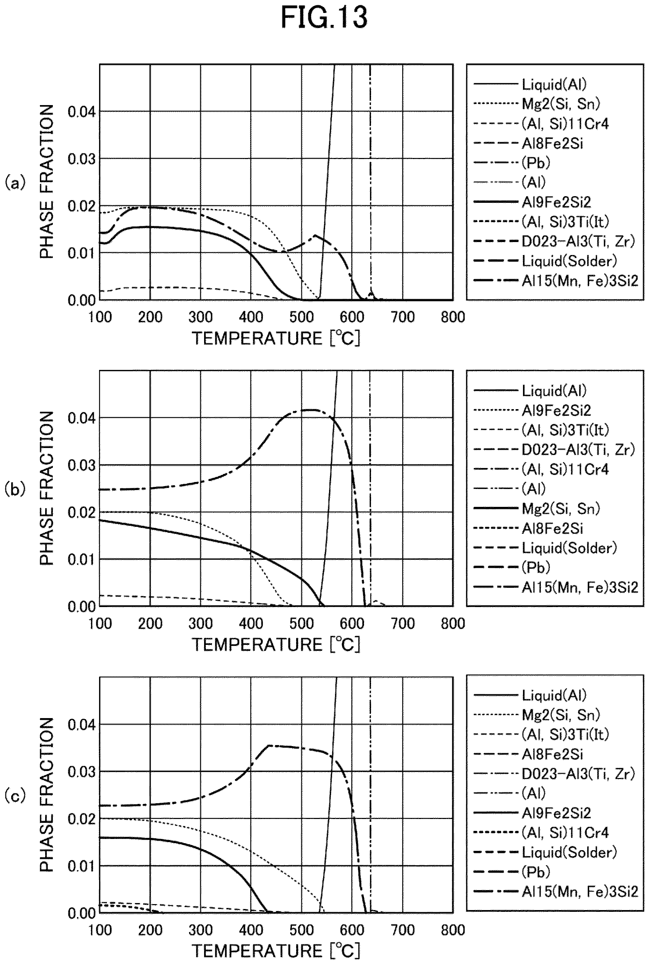

   FIG. 13 is a diagram for explaining prediction accuracy.
[FIG. 14]

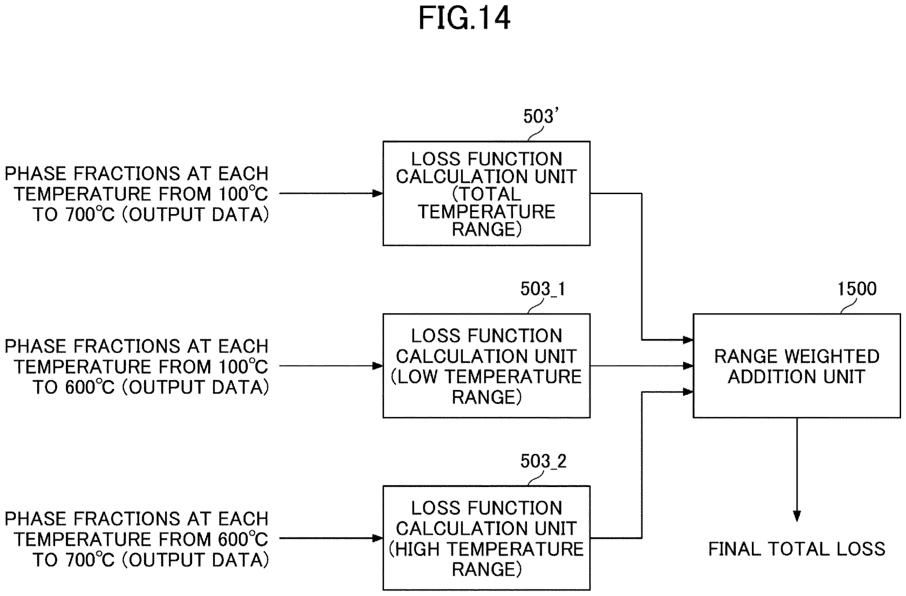

   FIG. 14 is a second diagram illustrating an example of the loss calculation method by the loss function calculation unit.

### Description of Embodiments

Hereinafter, each embodiment will be described with reference to the accompanying drawings. In the present specification and the drawings, components having substantially the same functional configuration are designated by the same reference numerals and redundant explanation will be omitted.

### [First Embodiment]

### <System Configuration of Prediction System, and Functional Configuration of Training apparatus and Prediction Apparatus>

First, a system configuration of a prediction system including a training apparatus and a prediction apparatus according to the first embodiment, and a functional configuration of the training apparatus and the prediction apparatus will be described. In the present embodiment, the prediction system uses a prediction model to predict a phase fraction at each temperature for a predetermined temperature range (a phase fraction at thermodynamic equilibrium; hereinafter referred to simply as "phase fraction") at low cost based on material composition information.

FIG. 1 is a diagram illustrating an example of a system configuration of the prediction system and an example of a functional configuration of the training apparatus and the prediction apparatus. As illustrated in FIG. 1, a prediction system 100 includes a training apparatus 110 and a prediction apparatus 120.

In the training apparatus 110, a training program is installed, and when the program is executed, the training apparatus 110 functions as a training data generation unit 111 and a training unit 112.

The training data generation unit 111 generates training data for training a prediction model and stores the training data in a training data storage 113. In the present embodiment, as the training data for training a prediction model, the training data storage 113 stores a plurality of pieces of material composition information with different combinations in association with the phase fractions at each temperature within a predetermined temperature range for the respective pieces of material composition information.

The training unit 112 reads the training data from the training data storage 113 and trains the prediction model using the read training data. The training unit 112 trains the prediction model so that the output data when the material composition information stored in the training data is input to the prediction model approaches the phase fraction at each temperature within a predetermined temperature range stored in association with the training data. Thus, the training unit 112 generates a trained prediction model. The training unit 112 stores the generated trained prediction model in the prediction unit 122 of the prediction apparatus 120.

The training unit 112 applies, as the prediction model, Seq2Seq with Attention mechanism or Transformer, which is an architecture (architecture commonly used for natural language processing, and the like) capable of calculating time series data that is data for each of predetermined time intervals. Thus, according to the prediction model, by inputting the material composition information stored in the training data, the output data corresponding to the phase fraction at each temperature within a predetermined temperature range is sequentially output.

Here, "the output data corresponding to the phase fraction at each temperature is sequentially output" means, for example, that the prediction model outputs output data corresponding to the phase fraction at the i+1-th temperature using the ground truth data for the temperature up to the i-th temperature stored in the training data. Note that i is an integer of 1 or more.

Thus, the training unit 112 has a configuration in which the output data corresponding to the phase fraction at each temperature is sequentially output (a configuration in which the output data corresponding to the phase fraction at each temperature is processed as time series data). Thus, according to the training unit 112, training can be performed that reflects the output data corresponding to the phase fraction in the adjacent temperature region.

A prediction program is installed in the prediction apparatus 120, and when the program is executed, the prediction apparatus 120 functions as a material composition input unit 121, a prediction unit 122, and a display unit 123.

When the material composition information of the material to be predicted is input, the material composition input unit 121 accepts the material composition information and notifies the prediction unit 122.

The prediction unit 122 includes a trained prediction model trained by the training unit 112, and by inputting the material composition information notified by the material composition input unit 121 into the trained prediction model, the phase fraction at each temperature within the predetermined temperature range is sequentially predicted. That is, the prediction unit 122 sequentially predicts the phase fraction at each predetermined temperature interval within the predetermined temperature range.

Here, "sequentially predicts the phase fraction at each predetermined temperature interval" means, for example, that the trained prediction model predicts the phase fraction at the i+1-th temperature using the phase fraction predicted by the trained prediction model for the temperature up to the i-th temperature (i is an integer of 1 or more).

Thus, the prediction unit 122 has a configuration in which the phase fraction at each temperature is sequentially predicted (that is, a configuration in which the phase fraction at each temperature is processed as time series data by a recurrent network). Thus, according to the prediction unit 122, prediction can be performed that reflects the predicted data of the phase fraction in the adjacent temperature region. As a result, for example, the deterioration of the prediction accuracy of the phase fraction can be prevented compared with the case where a multilayer neural network is applied (the case where the prediction result of the phase fraction in the adjacent temperature region is not reflected). That is, according to the prediction unit 122, the prediction accuracy can be improved in the prediction model that predicts the phase fraction over the predetermined temperature range based on the material composition.

The display unit 123 displays the phase fraction at each temperature within the predetermined temperature range predicted by the prediction unit 122. The display unit 123 displays the phase fraction at each temperature within the predetermined temperature range predicted by the prediction unit 122 in a color-coded manner for each phase.

### <Hardware Configuration of Training apparatus and Prediction Apparatus>

Next, the hardware configuration of the training apparatus 110 and the prediction apparatus 120 will be described. Because the training apparatus 110 and the prediction apparatus 120 have the same hardware configuration, the hardware configuration of the training apparatus 110 and the prediction apparatus 120 will be described collectively with reference to FIG. 2.

FIG. 2 is a diagram illustrating an example of the hardware configuration of the training apparatus and the prediction apparatus. As illustrated in FIG. 2, the training apparatus 110 and the prediction apparatus 120 include a processor 201, a memory 202, an auxiliary storage 203, an interface (I/F) device 204, a communication device 205, and a drive device 206. The respective hardware of the training apparatus 110 and the prediction apparatus 120 are connected to each other via a bus 207 .

The processor 201 includes various computing devices such as a central processing unit (CPU) and a graphics processing unit (GPU). The processor 201 reads and executes various programs (for example, a training program, a prediction program, and the like) on the memory 202.

The memory 202 includes a main storage device such as read only memory (ROM) and random access memory (RAM). The processor 201 and the memory 202 form what is called a computer, and the processor 201 executes the various programs read on the memory 202, thereby enabling the computer to perform various functions.

The auxiliary storage 203 stores various programs and various data used when the various programs are executed by the processor 201. For example, the training data storage 113 is implemented in the auxiliary storage 203.

The I/F device 204 is a connection device connected to an operating device 211 and a display device 212, which are examples of user interface devices. The communication device 205 is a communication device for communicating with an external device (not illustrated) via a network.

The drive device 206 is a device for setting a recording medium 213. The recording medium 213 here includes a medium that records information optically, electrically, or magnetically, such as a CD-ROM, a flexible disk, a magneto-optical disk, or the like. The recording medium 213 may also include a semiconductor memory that records information electrically, such as a ROM, a flash memory, or the like.

The various programs installed in the auxiliary storage 203 are installed, for example, when the distributed recording medium 213 is set in the drive device 206 and the various programs recorded in the recording medium 213 are read out by the drive device 206. Alternatively, the various programs installed in the auxiliary storage 203 may be installed by being downloaded from the network via the communication device 205.

### <Configuration of Training data, Input Data, and Ground truth data>

Next, a configuration of the training data and specific examples of the input data and the ground truth data included in the training data will be described. FIG. 3 is a diagram illustrating the configuration of the training data and the specific examples of the input data and the ground truth data.

As illustrated in FIG. 3, a training data 300 includes "input data" and "ground truth data" as information items.

In "input data", "material composition information 1", "material composition information 2", ..., and the like are stored that are the material composition information with different combinations. In FIG. 3, a reference numeral 301 denotes a specific example of "material composition information 1" and represents the weight percentage of each additive element constituting "6013", a designation of a known aluminum alloy standard.

Similarly, a reference numeral 302 denotes a specific example of "material composition information 2" and represents the weight percentage of each additive element constituting "6060", a designation of a known aluminum alloy standard.

In "ground truth data", "phase fraction 1", "phase fraction 2", ..., and the like are stored that are the phase fraction at each temperature in the predetermined temperature range (in the example of FIG. 3, 100°C to 700°C), associated with "material composition information 1", "material composition information 2", ..., and the like. In FIG. 3, a reference numeral 311 denotes a specific example of "phase fraction 1", and a reference numeral 312 denotes a specific example of "phase fraction 2". In both graphs, the horizontal axis represents the temperature and the vertical axis represents the phase fraction.

The reference numerals 311 and 312 are the results obtained by running a high-cost simulator with high prediction accuracy over a long period of time, and the results are used as the ground truth data in the present embodiment.

Here, a simulator refers to software that calculates the phase fraction at each temperature within a predetermined temperature range using thermodynamic equilibrium calculations. For example, the simulator includes software such as CaTCalc (registered trademark) and MatCalc (registered trademark). Alternatively, the simulator may include software such as Termosuite (registered trademark), FactStage (registered trademark), and Pandat (registered trademark). Alternatively, the simulator may include software such as MALT2 (registered trademark), Thermo-Calc (registered trademark), and OpenCalphad (registered trademark).

### <Details of Each Unit of Training apparatus>

Next, details of each unit (the training data generation unit 111 and the training unit 112) of the training apparatus 110 will be described.

### (1) Functional Configuration of Training data Generation Unit

First, a functional configuration of the training data generation unit 111 will be described. FIG. 4 is a diagram illustrating an example of the functional configuration of the training data generation unit.

As illustrated in FIG. 4, the training data generation unit 111 includes an element information input unit 401, a combination determination unit 402, a simulation unit 403, and a storage control unit 404.

The element information input unit 401 receives input, for example, on the type of the additive elements to be added when producing a specific alloy. The element information input unit 401 receives input of the upper limit and the lower limit of the additive amount (weight%) of each of the additive elements that has been input.

The combination determination unit 402 randomly selects the additive amount for each of the additive elements that has been input under the constraints of the upper limit and the lower limit, and determines a plurality of combinations of the additive amount of each of the additive elements. The combination determination unit 402 notifies the simulation unit 403 of pieces of the material composition information indicated by the determined combinations.

The simulation unit 403 calculates the phase fraction at each temperature within the predetermined temperature range for each piece of the material composition information notified by the combination determination unit 402, by running the above-mentioned simulator. The simulation unit 403 also notifies the storage control unit 404 of the pieces of the material composition information and the phase fraction at each temperature within the predetermined temperature range associated with the pieces of the material composition information.

The storage control unit 404 generates training data in which the pieces of the material composition information notified by the simulation unit 403 is "input data" and the phase fraction at each temperature within the corresponding predetermined temperature range associated with the pieces of the material composition information is "ground truth data", and stores the training data in the training data storage 113.

### (2) Functional Configuration of Training unit

Next, the functional configuration of the training unit 112 will be described. FIG. 5 is a diagram illustrating an example of the functional configuration of the training unit.

As illustrated in FIG. 5, the training unit 112 includes a prediction model and a loss function calculation unit 503.

As described above, Seq2Seq with Attention mechanism or Transformer is applied as the prediction model, and the prediction model includes an encoder 501 and a decoder 502.

The encoder 501 outputs a feature, upon the input of "material composition information 1", "material composition information 2", ..., and the like stored in "input data" of the training data 300.

The decoder 502 sequentially outputs output data upon the input of the feature output by the encoder 501. Specifically, the decoder 502 reads "phase fraction 1", "phase fraction 2", ..., and the like stored in "ground truth data" of the training data 300, and outputs the output data at the i+1-th temperature by using the phase fraction for the temperature up to the i-th temperature (that is, the ground truth data for the temperature up to the i-th temperature). Thus, the decoder 502 can sequentially output the output data corresponding to the phase fraction at each temperature within the predetermined temperature range based on the feature output from the encoder 501 when, for example, "material composition information 1" is input and based on "ground truth data" of "phase fraction 1". Similarly, the decoder 502 can sequentially output the output data corresponding to the phase fraction at each temperature within the predetermined temperature range based on the feature output from the encoder 501 when, for example, "material composition information 2" is input and based on "ground truth data" of "phase fraction 2".

In the output layer of the decoder 502, a softmax function is used. Accordingly, a plurality of values (that is, values of each phase) included in the output data of the decoder 502 are in the range of 0 to 1, and the sum (that is, the sum of the values of each phase) of the plurality of values is "1".

Thus, by using the softmax function in the output layer of the decoder 502, it is possible to sequentially output the output data corresponding to the phase fraction at each temperature within the predetermined temperature range without performing additional calculation.

The loss function calculation unit 503 reads "phase fraction 1", "phase fraction 2", ..., and the like stored in "ground truth data" of the training data 300 and compares them with the output data at the i+1-th temperature output by the decoder 502.

At the time of the comparison, the loss function calculation unit 503 calculates multiple types of losses and calculates a total loss by performing weighted addition of the calculated multiple types of losses. Further, the loss function calculation unit 503 updates model parameters of the encoder 501 and the decoder 502 based on the calculated total loss. As a result, a trained prediction model (including a trained encoder and a trained decoder) is generated.

### (3) Operation Example 1 of Training unit

Next, an operation example of the training unit 112 (mainly an operation example of the prediction model) will be described. FIG. 6 is a first diagram illustrating an operation example of the training unit.

FIG. 6(a) illustrates a state in which "input data" of the training data 300 is read and the read data is input to the encoder 501, and the feature is output from the encoder 501. FIG. 6(a) illustrates a state in which the decoder 502 outputs the output data when the start signal is input to the decoder 502. In the example of FIG. 6(a), the output data output by the decoder 502 corresponds to the phase fraction at 700°C. The start signal is a signal for starting the operation of the decoder 502, and in the present embodiment, an arbitrary value (for example, "000000", and the like) different from the output data output from the decoder 502 is input to the decoder 502 as the start signal.

FIG. 6(b) illustrates a state in which the decoder 502 outputs the output data when the feature output from the encoder 501 is input to the decoder 502 and the phase fraction at 700°C (the ground truth data at 700°C) is input to the decoder 502. In the example of FIG. 6(b), the output data output by the decoder 502 corresponds to the phase fraction at 690°C. The output data (here, the output data corresponding to the phase fraction at 690°C) output by the decoder 502 is notified to the loss function calculation unit 503.

FIG. 6(c) illustrates a state in which the decoder 502 outputs the output data when the feature output by the encoder 501 is input to the decoder 502 and the phase fraction up to 690°C (the ground truth data up to 690°C) is input to the decoder 502. However, in FIG. 6(c), only the ground truth data at 690°C is illustrated due to space constraints (in practice, the ground truth data at 700°C and the ground truth data at 690°C are input to the decoder 502 with weight applied thereto).

In the example of FIG. 6(c), the output data output by the decoder 502 corresponds to the phase fraction at 680°C. The output data (here, the output data corresponding to the phase fraction at 680°C) output by the decoder 502 is notified to the loss function calculation unit 503.

FIG. 6(d) illustrates a state in which the decoder 502 outputs the output data when the feature output by the encoder 501 is input to the decoder 502 and the phase fraction up to 120°C (the ground truth data up to 120°C) is input to the decoder 502. However, in FIG. 6(d), only the ground truth data at 120°C is illustrated due to space constraints (in practice, each ground truth data from 700°C to 120°C is input to the decoder 502 with weight applied thereto).

In the example of FIG. 6(d), the output data output by the decoder 502 corresponds to the phase fraction at 110°C. The output data (here, the output data corresponding to the phase fraction at 110°C) output by the decoder 502 is notified to the loss function calculation unit 503.

FIG. 6(e) illustrates a state in which the decoder 502 outputs output data when the feature output from the encoder 501 is input to the decoder 502 and the phase fraction up to 110°C (the ground truth data up to 110°C) is input to the decoder 502. However, in FIG. 6(e), only the ground truth data at 110°C is illustrated due to space constraints (in practice, each ground truth data from 700°C to 110°C is input to the decoder 502 with weight applied thereto).

In the example of FIG. 6(e), the output data output by the decoder 502 corresponds to the phase fraction at 100°C. The output data (here, the output data corresponding to the phase fraction at 100°C) output by the decoder 502 is notified to the loss function calculation unit 503.

### (4) Operation Example 2 of Training unit

Next, an operation example of the training unit 112 (mainly an operation example of the loss function calculation unit 503) will be described. FIG. 7 is a second diagram illustrating an operation example of the training unit. As illustrated in FIG. 7, the loss function calculation unit 503 reads "ground truth data" of the training data 300. The example of FIG. 7 illustrates a state in which the phase fraction at 700°C (the ground truth data); the phase fraction at 690 °C (the ground truth data); ...; and the phase fraction at 100°C (the ground truth data), that are the phase fraction at each temperature in the predetermined temperature range included in "phase fraction 1", are read.

The example of FIG. 7 illustrates a state in which, as a result of sequentially outputting the output data from the decoder 502 based on the feature output from the encoder 501 when "material composition information 1" is input, the phase fraction at 700°C (the output data); the phase fraction at 690°C (the output data); ...; and the phase fraction at 100°C (the output data), are retained in the loss function calculation unit 503.

The example of FIG. 7 illustrates a state in which the loss function calculation unit 503 calculates the total loss by comparing the phase fraction at each temperature from 100°C to 700°C (the ground truth data) with the phase fraction at each temperature from 100°C to 700°C (the output data). The example of FIG. 7 illustrates a state in which the loss function calculation unit 503 updates the model parameters of the encoder 501 and the decoder 502 based on the calculated total loss. The functional configuration of the loss function calculation unit 503 for calculating the total loss will be described in detail below.

### (5) Details of Functional Configuration of Loss Function Calculation Unit

FIG. 8 is a first diagram illustrating an example of a loss calculation method by the loss function calculation unit. As illustrated in FIG. 8, the loss function calculation unit 503 includes, as a function for calculating multiple types of losses, a formation/disappearance temperature phase fraction error calculation unit 801, a phase fraction error calculation unit 802, a phase fraction error (logarithmic value) calculation unit 803, a phase fraction error (differential value) calculation unit 804, a cross entropy error calculation unit 805, and a weighted addition unit 806.

The formation/disappearance temperature phase fraction error calculation unit 801 compares the phase fraction at each temperature from 100°C to 700°C (the ground truth data) with the phase fraction at each temperature from 100°C to 700°C (the output data) for the temperature at which the curve of each phase fraction becomes 0 among the ground truth data of the training data. Thus, the formation/disappearance temperature phase fraction error calculation unit 801 adds, for all phases, the error between the phase fraction specified based on the output data and the phase fraction specified based on the training data, at the temperature at which each phase is formed or disappeared, the temperature being specified based on the training data; and outputs the addition result (a first addition result).

The phase fraction error calculation unit 802 compares the phase fraction at each temperature from 100°C to 700°C (the ground truth data) with the phase fraction at each temperature from 100°C to 700°C (the output data). Thus, the phase fraction error calculation unit 802 adds, for the predetermined temperature range, the error of the phase fractions at each temperature; and outputs the addition result (a second addition result).

The phase fraction error (logarithmic value) calculation unit 803 compares the phase fraction at each temperature from 100°C to 700°C (the ground truth data) with the phase fraction at each temperature from 100°C to 700°C (the output data). Thus, the phase fraction error (logarithmic value) calculation unit 803 adds, for the predetermined temperature range, the error between the logarithmic values of the phase fraction at each temperature within the predetermined temperature range; and outputs the addition result (a third addition result).

The phase fraction error (differential value) calculation unit 804 compares the phase fraction at each temperature from 100°C to 700°C (the ground truth data) with the phase fraction at each temperature from 100°C to 700°C (the output data). Thus, the phase fraction error (differential value) calculation unit 804 adds, for the predetermined temperature range, the error between the differential values of the phase fractions between adjacent temperatures; and outputs the addition result (a fourth addition result).

The cross entropy error calculation unit 805 compares the phase fraction at each temperature from 100°C to 700°C (the ground truth data) with the phase fraction at each temperature from 100°C to 700°C (the output data). Thus, the cross entropy error calculation unit 805 adds, for the predetermined temperature range, the error between the ratios of the phase fractions at each temperature within the predetermined temperature range; and outputs the addition result (a fifth addition result).

The weighted addition unit 806 calculates the total loss by performing a weighted addition of the addition results (the first addition result to the fifth addition result) output from the formation/disappearance temperature phase fraction error calculation unit 801 to the cross entropy error calculation unit 805.

By calculating the multiple types of losses and performing the weighted addition, the loss function calculation unit 503 can process the loss between the output data and the ground truth data in a multifaceted manner. As a result, the training unit 112 can appropriately update the model parameters when training the prediction model.

### <Flow of Training process>

Next, a flow of a training process by the training apparatus 110 will be described. FIG. 9 is a flowchart illustrating the flow of the training process.

In step S901, the training data generation unit 111 receives input, as element information, on the type of the additive elements to be added when producing a specific alloy, and the upper limit and the lower limit of the additive amount (weight%) of each of the additive elements.

In step S902, the training data generation unit 111 randomly selects the additive amount for each of the additive elements under the constraints of the upper limit and the lower limit, and determines a plurality of combinations of the additive amount of each of the additive elements.

In step S903, the training data generation unit 111 calculates the phase fraction at each temperature within the predetermined temperature range for each piece of the material composition information indicated by the determined plurality of combinations, by running the simulator.

In step S904, the training data generation unit 111 generates the training data and stores the training data in the training data storage 113.

In step S905, the training unit 112 inputs a start signal to the decoder 502.

In step S906, the training unit 112 reads "input data" of the training data and inputs the read data to the prediction model.

In step S907, the training unit 112 sequentially inputs the phase fraction for the temperature up to the i-th temperature of "ground truth data" of the training data (that is, the ground truth data for the temperature up to the i-th temperature), and sequentially outputs the i+1-th output data.

In step S908, the training unit 112 determines whether all the output data corresponding to the phase fraction at each temperature within the predetermined temperature range is output. When it is determined in step S908 that there is a temperature for which the output data corresponding to the phase fraction is not output (NO in step S908), the process returns to step S907.

When it is determined in step S908 that all the output data corresponding to the phase fraction at each temperature within the predetermined temperature range is output (YES in step S908), the process proceeds to step S909.

In step S909, the training unit 112 reads "ground truth data" of the training data and compares the read data with the output data.

In step S910, the training unit 112 calculates the multiple types of losses and performs the weighted addition of the calculated multiple types of losses, and thereby calculates the total loss. The training unit 112 updates the model parameters of the encoder 501 and the decoder 502 based on the calculated total loss.

In step S911, the training unit 112 determines whether to continue the training process. When it is determined in step S911 that the training process is to be continued (YES in step S911), the process returns to step S906 and reads next "input data" of the training data to perform the similar process.

When it is determined in step S911 that the training process is to be terminated (NO in step S911), the process proceeds to step S912.

In step S912, the training unit 112 stores the trained encoder and the trained decoder as a trained prediction model in the prediction apparatus 120.

### <Details of Each Unit of Prediction Apparatus>

Next, details of each unit (here, the prediction unit 122) of the prediction apparatus 120 will be described.

### (1) Functional Configuration of Prediction Unit

First, the functional configuration of the prediction unit 122 will be described. FIG. 10 is a diagram illustrating an example of the functional configuration of the prediction unit.

As illustrated in FIG. 10, the prediction unit 122 includes a trained encoder 1001 and a trained decoder 1002 that are trained by the training unit 112. The trained encoder 1001 and the trained decoder 1002 form a trained prediction model.

When the material composition information (the material composition information of the material to be predicted) notified from the material composition input unit 121 is input, the trained encoder 1001 calculates the feature and outputs the calculated feature to the trained decoder 1002.

When the feature output from the trained encoder 1001 is input, the trained decoder 1002 sequentially outputs the predicted data. Specifically, the trained decoder 1002 outputs the predicted data at the i+1-th temperature using the predicted data for the temperature up to the i-th temperature. Thus, the trained decoder 1002 can predict the phase fraction at each temperature within the predetermined temperature range, based on the feature output from the trained encoder 1001 by inputting the material composition information of the material to be predicted.

### (2) Operation Example of Prediction Unit

Next, an operation example of the prediction unit 122 will be described. FIG. 11 is a diagram illustrating an operation example of the prediction unit. FIG. 11(a) illustrates a state in which the material composition information of the material to be predicted is input to the trained encoder 1001, and the feature is output from the trained encoder 1001. FIG. 11(a) illustrates a state in which the feature output from the trained encoder 1001 is input to the trained decoder 1002 and the start signal is input to the trained decoder 1002, and the predicted data (the phase fraction at 700°C) is output.

FIG. 11(b) illustrates a state in which the feature output from the trained encoder 1001 is input to the trained decoder 1002 and the predicted data (the phase fraction at 700°C) is input to the trained decoder 1002. Thus, the trained decoder 1002 outputs the predicted data (the phase fraction at 690°C) .

FIG. 11(c) illustrates a state in which the feature output from the trained encoder 1001 is input to the trained decoder 1002, and the predicted data (the phase fraction up to 690°C) is input to the trained decoder 1002. In FIG. 11(c), only the phase fraction at 690°C is illustrated due to space constraints (in practice, the phase fraction at 700°C and the phase fraction at 690°C are input to the trained decoder 1002 with weight applied thereto). Thus, the trained decoder 1002 outputs the predicted data (the phase fraction at 680°C).

FIG. 11(d) illustrates a state in which the feature output from the trained encoder 1001 is input to the trained decoder 1002, and the predicted data (the phase fraction up to 120°C) is input to the trained decoder 1002. In FIG. 11(d), only the phase fraction at 120°C is illustrated due to space constraints (in practice, each phase fraction from 700°C to 120°C is input to the trained decoder 1002 with weight applied thereto). Thus, the trained decoder 1002 outputs the predicted data (the phase fraction at 110°C).

FIG. 11(e) illustrates a state in which the feature output from the trained encoder 1001 is input to the trained decoder 1002, and the predicted data (the phase fraction up to 110°C) is input to the trained decoder 1002. In FIG. 11(e), only the phase fraction at 110°C is illustrated due to space constraints (in practice, each phase fraction from 700°C to 110°C is input to the trained decoder 1002 with weight applied thereto). Thus, the trained decoder 1002 outputs the predicted data (the phase fraction at 100°C). In the example illustrated in FIG. 11(e), the predicted data (the phase fraction at 100°C) output from the trained decoder 1002 when the feature and the predicted data (the phase fraction up to 110°C) are input is also illustrated together.

### <Flow of Prediction Process>

Next, a flow of a prediction process by the prediction apparatus 120 will be described. FIG. 12 is a flowchart illustrating the flow of the prediction process.

In step S1201, the material composition input unit 121 receives input of the material composition information of the material to be predicted.

In step S1202, the prediction unit 122 receives the start signal and inputs the input material composition information into the trained prediction model to predict the phase fraction at each temperature within the predetermined temperature range.

In step S1203, the display unit 123 displays the predicted phase fraction at each temperature.

### <Prediction Accuracy>

Next, the prediction accuracy of the phase fraction at each temperature within the predetermined temperature range predicted by the prediction unit 122 will be described. FIGS. 13A to 13C are diagrams for explaining the prediction accuracy. FIGS. 13A to 13C illustrate the predicted data when the weight percentage of each additive element constituting "6013", which is a designation of a known aluminum alloy standard, is input as the material composition information of the material to be predicted. In the present embodiment, when evaluating the prediction accuracy, the ground truth data denoted by the reference numeral 311 in FIG. 3 is used. Mean Squared Logarithmic Error (MSLE) loss was used to calculate the loss with respect to the ground truth data.

FIG. 13(a) illustrates the predicted data when a fully-connected multilayer neural network is applied to the prediction model, as a comparative example. In FIG. 13(a), the MSLE loss with respect to the ground truth data is 3.79×10⁻⁴.

FIG. 13(b) illustrates the predicted data when Seq2Seq with Attention mechanism is applied to the prediction model. In FIG. 13(b), the MSLE loss with respect to the ground truth data is 5.1×10⁻⁵.

FIG. 13(c) illustrates the predicted data when Transformer is applied to the prediction model. In FIG. 13(c), the MSLE loss with respect to the ground truth data is 2.45×10⁻⁵.

Thus, when the fully-connected multilayer neural network is applied as the prediction model to realize the calculation of the phase fraction at low cost, the estimation accuracy is low in some temperature regions (for example, 400°C to 600°C).

In contrast, when Seq2Seq with Attention mechanism is applied as the prediction model to realize the calculation of the phase fraction at low cost, the loss is significantly improved. In addition, when Transformer is applied, the phase fraction of approximately the same level as the ground truth data is reproduced.

In other words, when predicting the phase fraction over the predetermined temperature range based on the material composition information, the prediction accuracy can be improved by performing the following. The phase fraction at each temperature is processed as time series data by using a recurrent network; and the loss is processed in a multifaceted manner by using multiple types of loss functions.

### <Summary>

As is clear from the above description, the prediction apparatus 120 according to the first embodiment includes: a trained model trained by using training data in which the material composition of the material to be learned is associated with the phase fraction of the material to be learned at each temperature within the predetermined temperature range, the trained model being configured to predict the phase fraction at the i+1-th temperature by using the phase fraction predicted by the trained model for the temperature up to the i-th temperature within the predetermined temperature range, wherein the prediction apparatus 120 is configured to input the material composition of the material to be predicted into the trained model, thereby predicting the phase fraction of the material to be predicted at each temperature within the predetermined temperature range.

Thus, according to the first embodiment, the prediction accuracy can be improved in the prediction model that predicts the phase fraction over the predetermined temperature range based on the material composition.

### [Second Embodiment]

In the first embodiment described above, the total loss was calculated by performing the weight addition of the multiple types of losses calculated for the predetermined temperature range. However, the calculation method of the total loss is not limited thereto. For example, a final total loss may be calculated by dividing the predetermined temperature range into a plurality of temperature ranges and further performing the weight addition of the total loss calculated in each temperature range.

FIG. 14 is a second diagram illustrating an example of a loss calculation method by the loss function calculation unit. The difference with the loss function calculation unit 503 illustrated in FIG. 5 is that the loss function calculation unit 503 functions as a loss function calculation unit (total temperature range) 503', and that the loss function calculation unit 503 includes, in addition to the loss function calculation unit (total temperature range) 503', a loss function calculation unit (low temperature range) 503_1; a loss function calculation unit (high temperature range) 503_2; and a range weighted addition unit 1500.

The loss function calculation unit (total temperature range) 503' reads "phase fraction 1", "phase fraction 2", ..., and the like stored in "ground truth data" of the training data 300. The loss function calculation unit (total temperature range) 503' acquires output data corresponding to the phase fraction at each temperature from 100°C to 700°C output by the decoder 502. In addition, the loss function calculation unit (total temperature range) 503' compares the ground truth data with the output data for the predetermined temperature range, and outputs a total loss (total temperature range). Although not illustrated in the example of FIG. 14, the loss function calculation unit (total temperature range) 503' includes the units illustrated in FIG. 8, and when the units operate, the total loss (total temperature range) is output.

The loss function calculation unit (low temperature range) 503_1 reads the data of the low temperature range (the temperature range of 100°C to 600°C) among "phase fraction 1", "phase fraction 2", ..., and the like stored in "ground truth data" of the training data 300. The loss function calculation unit (low temperature range) 503_1 acquires the output data corresponding to the phase fraction at each temperature from 100°C to 600°C among the output data output by the decoder 502. Further, the loss function calculation unit (low temperature range) 503_1 compares the ground truth data with the output data for the low temperature range of 100°C to 600°C, and outputs the total loss (low temperature range). Although not illustrated in the example of FIG. 14, the loss function calculation unit (low temperature range) 503_1 includes the units illustrated in FIG. 8, and when the units operate, the total loss (low temperature range) is output.

The loss function calculation unit (high temperature range) 503_2 reads the data of the high temperature range (the temperature range of 600°C to 700°C) among "phase fraction 1", "phase fraction 2", ..., and the like stored in "ground truth data" of the training data 300. The loss function calculation unit (high temperature range) 503_2 acquires the output data corresponding to the phase fraction at each temperature from 600°C to 700°C among the output data output by the decoder 502. Further, the loss function calculation unit (high temperature range) 503_2 compares the ground truth data with the output data for the high temperature range of 600°C to 700°C, and outputs the total loss (high temperature range). Although not illustrated in the example of FIG. 14, the loss function calculation unit (high temperature range) 503_2 includes the units illustrated in FIG. 8, and when the units operate, the total loss (high temperature range) is output.

The range weighted addition unit 1500 outputs the final total loss by performing the weighted addition of the total loss (total temperature range) output from the loss function calculation unit (total temperature range) 503'; the total loss (low temperature range) output from the loss function calculation unit (low temperature range) 503_1; and the total loss (high temperature range) output from the loss function calculation unit (high temperature range) 503_2.

As is clear from the above description, in the second embodiment, the total losses are calculated separately for each of the temperature ranges, and the final total loss obtained by performing the weighted addition of the calculated total losses is used for training. Thus, according to the second embodiment, even in the case where the phase fraction changes significantly in a narrow temperature range, the change can be accurately regarded as a loss and reflected in the training. As a result, according to the second embodiment, the prediction accuracy can be further improved in the prediction model for predicting the phase fraction over the predetermined temperature range based on the material composition.

### [Third Embodiment]

In each of the embodiments described above, the phase fraction error (logarithmic value) calculation unit 803 compares the phase fraction at each temperature from 100°C to 700°C (the ground truth data) with the phase fraction at each temperature from 100°C to 700°C (the output data), and calculates the error between the logarithmic values of the phase fraction at each temperature. However, the calculation method of the error between the logarithmic values by the phase fraction error (logarithmic value) calculation unit 803 is not limited thereto. For example, when calculating the logarithmic value of the phase fraction, a value according to the decimal place of the phase fraction may be added, thereby making the logarithmic value of the phase fraction non-negative, and then the error may be calculated.

In the second embodiment, the loss function calculation unit (total temperature range), the loss function calculation unit (low temperature range), and the loss function calculation unit (high temperature range) are provided to calculate each of the total losses. However, the calculation method of each of the total losses is not limited to this. For example, a loss function calculation unit (total temperature range), a loss function calculation unit (specific range), and a loss function calculation unit (non-specific range) may be provided to calculate each of the total losses.

The specific range may be, for example, the high temperature range or a temperature range different from the high temperature range (for example, a temperature range including a plurality of phase formations and phase disappearances). The non-specific range may be a temperature range excluding the specific range, for example, the low temperature range or a temperature range different from the low temperature range.

By providing the specific range in this manner, it is possible to, in updating the model parameters, reflect the errors that occur only in a part of the temperature region of the predetermined temperature range, which would otherwise not be noticeable when averaged over the predetermined temperature range, for example.

In the above embodiments, the prediction direction in the case of "sequentially predict the phase fraction at each predetermined temperature interval" is not specifically described, but the prediction direction may be either a direction of increasing the temperature or a direction of decreasing the temperature. Alternatively, the prediction direction may be both the direction of increasing the temperature and the direction of decreasing the temperature.

In the above embodiments, as a specific example of the material composition, an alloy composition indicating a ratio of the additive element (other metallic element or non-metallic element) to the metallic element contained in the alloy has been described. However, the material composition is not limited to the alloy composition. For example, a chemical composition indicating a ratio of each chemical component other than the alloy contained in a material, may be used.

In the above embodiments, as the trained prediction model, either Seq2Seq with Attention mechanism or Transformer is applied. However, the trained prediction model is not limited to these, and other architectures may be applied as long as they are capable of calculating time series data that is data for each of predetermined time intervals. Specifically, recurrent neural network (RNN), Bidirectional RNN, Seq2Seq, and the like may be applied. Alternatively, gated recurrent unit (GRU), long short term memory (LSTM), and the like may be applied.

In the above embodiments, the training apparatus 110 and the prediction apparatus 120 are configured separately, but the training apparatus 110 and the prediction apparatus 120 may be configured integrally.

In the above embodiments, usage scenarios for the phase fraction over the predetermined temperature range predicted based on the material composition is not described, but the predicted phase fraction may be used, for example, to search for a material composition with a target phase fraction. Accordingly, a material having a target phase fraction can be designed and developed.

The present invention is not limited to the configuration illustrated here, such as the configuration illustrated in the above embodiment or the combination with other elements. These aspects can be modified to the extent that they do not depart from the object of the present invention, and can be appropriately determined according to the application form.

The present application claims priority to Japanese Patent Application No. 2021-189833, filed November 24, 2021, with the Japanese Patent Office, the contents of which are incorporated herein by reference in their entirety.

### Description of the Reference Numerals

- 100 :: Prediction system
- 110 :: Training apparatus
- 111 :: Training data generation unit
- 112 :: Training unit
- 120 :: Prediction apparatus
- 121 :: Material composition input unit
- 122 :: Prediction unit
- 123 :: Display unit
- 300 :: Training data
- 401 :: Element information input unit
- 402 :: Combination determination unit
- 403 :: Simulation unit
- 404 :: Storage control unit
- 501 :: Encoder
- 502 :: Decoder
- 503 :: Loss function calculation unit
- 503' :: Loss function calculation unit (total temperature range)
- 503_1 :: Loss function calculation unit (low temperature range)
- 503_2 :: Loss function calculation unit (high temperature range)
- 801 :: Formation/disappearance temperature phase fraction error calculation unit
- 802 :: Phase fraction error calculation unit
- 803 :: Phase fraction error (logarithmic value) calculation unit
- 804 :: Phase fraction error (differential value) calculation unit
- 805 :: Cross entropy error calculation unit
- 806 :: Weighted addition unit
- 1001 :: Trained encoder
- 1002 :: Trained decoder
- 1500 :: Range weighted addition unit

## Claims

1. A prediction apparatus comprising:
a trained model trained by using training data in which a material composition of a material to be learned is associated with a phase fraction of the material to be learned at each temperature within a predetermined temperature range, the trained model being configured to predict a phase fraction at an i+1-th temperature by using a phase fraction predicted by the trained model for one or more temperatures up to an i-th temperature within the predetermined temperature range (where i is an integer of 1 or more), wherein
the prediction apparatus is configured to input a material composition of a material to be predicted into the trained model, thereby predicting a phase fraction of the material to be predicted at each temperature within the predetermined temperature range.

2. The prediction apparatus according to claim 1, wherein the trained model is applied with an architecture capable of calculating time series data that is data for each of predetermined time intervals, and the trained model predicts a phase fraction for each of predetermined temperature intervals based on the material composition of the material to be predicted.

3. The prediction apparatus according to claim 2, wherein the trained model is any one of RNN, Bidirectional RNN, Seq2Seq, Seq2Seq with Attention mechanism, GRU, LSTM, or Transformer.

4. The prediction apparatus according to claim 3, wherein the trained model includes:
an encoder configured to output a feature, upon input of the material composition of the material to be predicted; and
a decoder configured to predict the phase fraction at the i+1-th temperature, upon input of the output feature and the phase fraction predicted for the temperature up to the i-th temperature.

5. The prediction apparatus according to claim 1, wherein the phase fraction is a phase fraction at thermodynamic equilibrium.

6. A training apparatus comprising:
a model trained by using training data in which a material composition of a material to be learned is associated with a phase fraction of the material to be learned at each temperature within a predetermined temperature range, the model being configured to output output data corresponding to a phase fraction at an i+1-th temperature by using ground truth data for one or more temperatures up to an i-th temperature within the predetermined temperature range (where i is an integer of 1 or more).

7. The training apparatus according to claim 6, wherein the model includes:
an encoder configured to output a feature, upon input of the material composition of the material to be learned; and
a decoder configured to output the output data corresponding to the phase fraction at the i+1-th temperature, upon input of the output feature and the ground truth data of the phase fraction for the temperature up to the i-th temperature.

8. The training apparatus according to claim 6, further comprising a calculation unit configured to, upon input of the material composition of the material to be learned, compare the output data output by the model with the phase fraction at each temperature within the predetermined temperature range that is associated with the material composition of the material to be learned, thereby calculating a loss function.

9. The training apparatus according to claim 8, wherein the calculation unit is configured to calculate the loss function and to output a loss, and the loss includes at least any one of:
a first addition result obtained by adding, for all phases, an error between a phase fraction specified based on the output data and a phase fraction specified based on the training data, at a temperature at which each phase is formed or disappeared, the temperature being specified based on the training data;
a second addition result obtained by adding, for the predetermined temperature range, an error between a phase fraction at each temperature included in the output data and a phase fraction at each temperature included in the training data;
a third addition result obtained by adding, for the predetermined temperature range, an error between a logarithmic value of the phase fraction at each temperature included in the output data and a logarithmic value of the phase fraction at each temperature included in the training data;
a fourth addition result obtained by adding, for the predetermined temperature range, an error between a differential value of phase fractions between adjacent temperatures among a group of the phase fraction at each temperature included in the output data and a differential value of the phase fractions between adjacent temperatures among a group of the phase fraction at each temperature included in the training data; or
a fifth addition result obtained by adding, for the predetermined temperature range, an error between a ratio of the phase fraction at each temperature included in the output data and a ratio of the phase fraction at each temperature included in the training data.

10. The training apparatus according to claim 9, wherein the calculation unit is configured to perform a weighted addition of the first addition result to the fifth addition result.

11. The training apparatus according to claim 9, wherein when calculating the logarithmic value of the phase fraction, the calculation unit is configured to add a value according to a decimal place of the phase fraction, thereby making the logarithmic value of the phase fraction non-negative.

12. The training apparatus according to claim 8, wherein the calculation unit is configured to divide the predetermined temperature range into a specific range including a plurality of phase formations and a plurality of phase disappearances and into a non-specific range, which is a range excluding the specific range, thereby calculating a loss function for the specific range and a loss function for the non-specific range separately.

13. The training apparatus according to claim 6, wherein the material composition of the material to be learned included in the training data is determined by an amount selected at random between a lower limit and an upper limit of an amount of each element.

14. The training apparatus according to claim 6, wherein the material composition includes any one of a chemical composition indicating a ratio of each chemical component contained in a material; or an alloy composition indicating a ratio of each metallic element or each non-metallic element included in an alloy.

15. A prediction method comprising:
executing, by a computer, a trained model trained by using training data in which a material composition of a material to be learned is associated with a phase fraction of the material to be learned at each temperature within a predetermined temperature range, the trained model being configured to predict a phase fraction at an i+1-th temperature by using a phase fraction predicted by the trained model for one or more temperatures up to an i-th temperature within the predetermined temperature range (where i is an integer of 1 or more), wherein
a material composition of a material to be predicted is input into the trained model, thereby a phase fraction of the material to be predicted is predicted at each temperature within the predetermined temperature range.

16. A training method comprising:
executing, by a computer, a model trained by using training data in which a material composition of a material to be learned is associated with a phase fraction of the material to be learned at each temperature within a predetermined temperature range, the model being configured to output output data corresponding to a phase fraction at an i+1-th temperature by using ground truth data for one or more temperatures up to an i-th temperature within the predetermined temperature range (where i is an integer of 1 or more).

17. A prediction program for causing a computer to execute a trained model trained by using training data in which a material composition of a material to be learned is associated with a phase fraction of the material to be learned at each temperature within a predetermined temperature range, the trained model being configured to predict a phase fraction at an i+1-th temperature by using a phase fraction predicted by the trained model for one or more temperatures up to an i-th temperature within the predetermined temperature range (where i is an integer of 1 or more), wherein
a material composition of a material to be predicted is input into the trained model, thereby a phase fraction of the material to be predicted is predicted at each temperature within the predetermined temperature range.

18. A training program for causing a computer to execute a model trained by using training data in which a material composition of a material to be learned is associated with a phase fraction of the material to be learned at each temperature within a predetermined temperature range, the model being configured to output output data corresponding to a phase fraction at an i+1-th temperature by using ground truth data for one or more temperatures up to an i-th temperature within the predetermined temperature range (where i is an integer of 1 or more).
